# EUROPÄISCHE PATENTANMELDUNG

(11) **EP 2 161 045 A2**
(43) Veröffentlichungstag der Anmeldung: **10.03.2010**
(21) Anmeldenummer: 09166092.8
(22) Anmeldetag: 22.07.2009
(51) Int. Cl.: A61L 31/02, A61L 31/14

(54) **Verwendung von organischen Gold-Komplexen als bioaktive und radioopaque Stentbeschichtung für permanente und degradierbare vaskuläre Implantate**

(30) Priorität: 19.08.2008 DE 102008038368
(71) Anmelder: Biotronik VI Patent AG, 6341 Baar (CH)
(72) Erfinder: Wittchow, Eric, 90403, Nürnberg (DE)
(74) Vertreter: Lindner-Vogt, Karin L.

(57) **Zusammenfassung**

Die Erfindung betrifft einen Stent mit einer Beschichtung oder Kavitätenfüllung bestehend aus oder enthaltend einen organischen Au-Komplex.

## Beschreibung

Die Erfindung betrifft einen mit einem bioaktiven und gleichzeitig radioopaquen Material beschichteten Stent und die Verwendung zur Herstellung derartiger Stents.

### Technologischer Hintergrund und Stand der Technik

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper, durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Der Vorgang der Positionierung und Expansion des Stents während der Prozedur und die abschließende Lage des Stents im Gewebe nach Beendigung der Prozedur muss durch den Kardiologen überwacht werden. Dies kann durch bildgebende Verfahren, wie z.B. durch Röntgenuntersuchungen erfolgen.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe für Stents umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen, L605), Reintitan und Titanlegierungen (z.B. cp Titan, TiAl6V4 oder TiAl6Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Eine biologische Reaktion auf polymere, keramische oder metallische Implantatwerkstoffe hängt von der Konzentration, Einwirkdauer und Art der Zuführung ab. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Bekannt ist, dass sich ein höheres Maß an Biokompatibilität und damit eine Verbesserung der Restenoserate erreichen lässt, wenn Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Weitere Strategien zur Vermeidung der Restenose konzentrieren sich darauf, die Proliferation durch Medikation, z. B. Behandlung mit Cytostatika, zu hemmen. Die Wirkstoffe können zum Beispiel auf der Implantatoberfläche in Form einer Beschichtung bereitgestellt werden.

Trotz der erreichten Fortschritte besteht noch immer ein hohes Bedürfnis, eine bessere Integration des Stents in sein biologisches Umfeld zu erreichen und dadurch die Restenoserate zu senken und gleichzeitig eine ausreichende Darstellbarkeit des Stents während der Applikation und auch danach zu gewährleisten.

### Erfindungsgemäße Lösung

Die Aufgabe wird erfindungsgemäß durch Bereitstellung eines Stents mit einer Beschichtung oder Kavitätenfüllung gelöst, die aus einem organischen Gold [Au]-Komplex besteht oder diesen enthält.

Der Erfindung liegt die Erkenntnis zugrunde, dass Gold in Form von Komplexen, insbesondere der Gold-Ionen Au(I) und Au(III), sowohl als radioopaquer Marker für die Röntgenbildgebung, als auch als restenosehemmende bzw. -vorbeugende Substanz wirkt. Damit sind zwei wesentliche Eigenschaften eines beschichteten Stent, die Restenosevermeidung und die bildgebende Darstellbarkeit des Stents im Gewebe in einer einzigen Substanz vereint. In der Folge lassen sich die Herstell- und Beschichtungsverfahren wesentlich vereinfachen. Zum einen lässt sich die Anzahl der Arbeitsschritte zur Herstellung des Stents verringern, da die Herstellung von Marker und aktiver Beschichtung / Kavitätenfüllung zusammengefasst ist. Zum anderen erfolgt eine Vereinfachung gegenüber herkömmlichen Beschichtungen / Kavitätenfüllungen, die Marker als auch biologisch aktive Substanz enthalten; durch die Zusammenfassung dieser Komponenten können Unvereinbarkeiten von Marker und aktiver Substanz, zum Beispiel unterschiedliche Löslichkeiten, vermieden werden und eine Optimierung des Systems mit Hinsicht auf die angestrebte Applikation ist erleichtert.

Gemäß einer ersten Variante weist der Grundkörper des Stent demnach eine Beschichtung auf, die den erfindungsgemäßen Au-Komplex enthält oder aus diesem besteht. Eine Beschichtung im Sinne der Erfindung ist eine zumindest abschnittsweise Auftragung der Komponenten der Beschichtung auf den Grundkörper des Stents. Vorzugsweise wird die gesamte Oberfläche des Grundkörpers des Stents von der Beschichtung bedeckt. Eine Schichtdicke liegt vorzugsweise im Bereich von 1 nm bis 100 µm, besonders bevorzugt 300 nm bis 15 µm. Der Gewichtsanteil des Au-Komplexes an den die Beschichtung bildenden Komponenten der Beschichtung beträgt vorzugsweise mindestens 40%, besonders bevorzugt mindestens 70%. Die Beschichtung kann direkt auf die Implantatoberfläche aufgetragen werden. Die Verarbeitung kann nach Standardverfahren für die Beschichtung erfolgen. Es können einschichtige, aber auch mehrschichtige Systeme (zum Beispiel so genannte base coat -, drug coat - oder top coat - Schichten) erstellt werden. Die Beschichtung kann unmittelbar auf dem Grundkörper des Stents aufgebracht sein oder es sind weitere Schichten dazwischen vorgesehen, die beispielsweise der Haftvermittlung dienen.

Alternativ kann der Au-Komplex als Kavitätenfüllung vorliegen oder Bestandteil einer Kavitätenfüllung sein. Der Stent weist zu diesem Zwecke eine oder mehrere Kavitäten auf. Kavitäten befinden sich beispielsweise an der Oberfläche des Stents und lassen sich durch Laserablation in Mikrometerdimensionen erstellen. Bei Stents mit einem biodegradierbaren Grundkörper kann eine Kavität auch im Innern des Grundkörpers angeordnet sein, so dass die Freisetzung des Material erst nach Freilegung erfolgt. Der Fachmann kann sich bei der Ausgestaltung der Kavität an dem im Stand der Technik beschriebenen Systemen orientieren.

Neben der Verwendung erfindungsgemäßer Au-Komplexe kann die Beschichtung oder Kavitätenfüllung weitere Bestandteile enthalten, insbesondere eine polymere organische Matrix, in die der Au-Komplex in fein dispergierter Form eingebettet ist. Mit anderen Worten, der Stent weist eine Beschichtung oder Kavitätenfüllung auf, die aus einer polymeren Trägermatrix mit eingebettetem Au-Komplex besteht oder diese Komponenten enthält. Die Trägermatrix kann insbesondere weitere pharmazeutische Wirkstoffe, weitere Röntgenmarker oder auch Magnetresonanzmarker enthalten.

Wegen seines hohen Atomgewichts von 197 ist Gold als Röntgenmarker geeignet und auch vielfach als solcher eingesetzt worden.

Nach einer bevorzugten Ausführungsform der Erfindung ist der Au-Komplex ein antikanzerogener oder immunmodulatorischer Wirkstoff. So werden Au-Komplexe, wie z.B. Tauredon®, in der Behandlung von entzündlichen Erkrankungen, wie der rheumatoiden Arthritis eingesetzt. Solche Au-Komplexe haben also immunmodulatorische Wirkungen. Seit kurzem werden Au-Komplexe auch als geeignet zur Verwendung in der Tumortherapie beschrieben (I. Kostova, Anti-Cancer Agents in Medicinal Chemistry, 2006, 6, 19-32). Solche Au-Komplexe haben also antikanzerogene Wirkungen. Wirkstoffe mit antikanzerogener oder immunmodulatorischer Wirkung sind derzeit von besonderem Interesse als Mittel zur Restenoseprophylaxe. Besonders bevorzugt ist der Wirkstoff ausgewählt aus der Gruppe umfassend Aurothiomalat, Aurothioglucose, bis(Thiosulfat) Au(I), Thiopropansulfat-S-Au(I), Thiopropanolsulfonat-S-Au(I), 1,2-bis(Diphenylphosphin)ethan Au(I), 1,2-bis(Dipyridylphosphino)ethan Au(I) und tetrakis((tris(Hydroxymethyl)) phosphin)Au(I). Des Weiteren sind besonders bevorzugt Verbindungen, bei denen das Au(I) sowohl von einem Phosphoratom, als auch von einem Schwefelatom komplexiert wird, der Komplex also die Struktureinheit P-Au-S enthält. Ein Beispiel für diese Verbindungsklasse ist das Tetraacetylthioglucose-Au(I)-triethylposphin, welches auch unter dem Namen Auranofin bekannt ist.

Erfindungsgemäße Au-Komplexe sind bevorzugt Komplexe aus Gold-Ionen, wie Au(I) und Au(III). In bevorzugten Au-Komplexen bildet das Gold-Ion mit mindestens einem oder mehreren Elementen der V. und VI. Hauptgruppe des Periodensystems (IUPAC-Gruppen 15 und 16) der organischen Verbindung einen stabilen Komplex, wobei in einem Komplex unterschiedliche Elemente an der Komplexbildung beteiligt sein können (heterogener Komplex).

Wenn der Au-Komplex ein Au(I)-Komplex ist, so weist dieser vorzugsweise einen oder mehrere Schwefelliganden auf. Dies ist insbesondere der Thiolatligand, der Thiosulfatligand, der Disulfidligand und der Thiokohlenhydratligand (z.B. Thioglucose).

Ebenso bevorzugt sind Au(I)-Komplexe, die einen oder mehrere tertiäre Phosphinliganden aufweisen. Insbesondere ist der Phosphinligand ausgewählt aus der Gruppe umfassend Triethylphosphin, 1,2-bis(Diphenylphosphin)ethan, 1,2-bis(Dipyridylphosphin)ethan, und tris(Hydroxymethyl)phosphin.

Wenn der Au-Komplex ein Au(III)-Komplex ist, so weist dieser vorzugsweise einen oder mehrere mehrzähnige N-haltige Liganden auf. Insbesondere ist der N-haltige Ligand ausgewählt aus der Gruppe umfassend Ethylendiamin, Diethylendiamin, N-Benzyl-N,N-dimethylamin; aus Bispyridyl-Liganden, insbesondere (Bispyridyl)(OH)₂, (6-(1,1-Dimethylbenzyl)-2,2'-bipyridin-H)(OH); oder aus Trichlor(2-pyridylmethanol), Dichlor(N-ethylsalicylaldiminat), Trichlor(diethylendiamin), und Trichlor(bisethylen-diamin). Besonders bevorzugt sind Dithiocarbamatliganden.

Weitere, für die Zwecke der Erfindung geeignete Au-Komplexe sind in der Literatur beschrieben und Wege zur Herstellung der erfindungsgemäßen Au-Komplexe sind dem Fachmann bekannt. Beispielhaft sei auf einige einschlägige Veröffentlichungen hingewiesen, in denen auch auf antikanzerogene Eigenschaften der Verbindungen eingegangen wird:
- Kostova, Anti-Cancer Agents in Medicinal Chemistry, 2006, 6, 19-32.
- Milacic V.; Histol. Histopathol. 2008, 23, 101-108.
- Best S.L., Sadler P.J.; Gold Bulletin, 1996, 29, 87.
- Fricker S.P.; Gold Bulletin, 1996, 29, 53.
- Parish R.V. Cottrill S.M.; Gold Bulletin 1987, 20, 3.
- Tiekink E.R.T.; Gold Bulletin 2003, 36, 117.
- Mazid M.A. et al., J. Chem. Soc. Chem. Commun., 1980, 1261.
- Elder R.C. et al., J. Am. Chem. Soc., 1985, 107, 5024.
- McKeage M.J. et al., J. Coord. Chem. Rev., 2002, 232, 127.
- McKeage M.J. et al., Cancer Chemother. Pharmacol., 2000, 46, 343.
- Caruso F. et al., J. Med. Chem., 2003, 46, 1737.
- Pillarsetty N. et al., J. Med. Chem., 2003, 46, 1130.
- Buckley R.G. et al., 1996, 39, 5208.
- Coronello M. et al., Oncol. Res., 2000, 12, 361.
- Bruni B. et al., Croatica Chemica Acta, 1999, 72, 221.
Der Fachmann kann sich bei der Herstellung der Au-Komplexe an den Herstellungswegen dieser Veröffentlichungen orientieren.

Ist der Stent ganz oder in Teilen aus einem biokorrodierbaren metallischen Werkstoff geformt, insbesondere einer Magnesiumlegierung, so bestehen besondere Anforderungen an den Marker. Elementares Gold ist für diese Werkstoffe nicht geeignet, da es zur Lokalelementbildung mit umliegenden unedlen Metallen, wie insbesondere Magnesium, neigt, wodurch die Auflösung des unedleren Metalls stark beschleunigt wird. Durch die starke Komplexbildung in organischen Gold-Komplexen ist die Gefahr einer Reduzierung des Gold-Ions zu elementarem Gold und einer Lokalelementbildung gemindert oder günstigstenfalls völlig behoben, so dass der Einsatz der Au-Komplexe bei Stents aus einem biokorrodierbaren metallischen Werkstoff besonders bevorzugt ist. Vorzugsweise besteht der metallische Grundkörper aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung. Insbesondere ist der biokorrodierbare metallische Werkstoff eine Magnesiumlegierung.

Als biokorrodierbar im Sinne der Erfindung werden Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Unter Magnesiumlegierung, Eisenlegierung, Zinklegierung, Molybdänlegierung oder Wolframlegierung wird vorliegend ein metallisches Gefüge verstanden, dessen Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Die Beschichtung der erfindungsgemäßen Stents kann einen oder mehrere verschiedene Au(I)- oder Au(III)-Komplexe enthalten oder ggf. aus einer solchen Mischung bestehen.

Die Erfindung bezieht sich auch auf eine Verwendung von organischen Au-Komplexen zur Beschichtung oder Kavitätenfüllung von Stents.

Nachfolgend wird die Erfindung anhand eines Ausführungsbeispiels näher erläutert.

### Ausführungsbeispiel 1 - Beschichtung eines Stents

Ein Stent aus der biokorrodierbaren Magnesiumlegierung WE43 (97 Gew.% Magnesium, 4 Gew.% Yttrium, 3 Gew.% Seltenerdmetalle außer Yttrium) wird wie folgt beschichtet:

Es wird eine Lösung von Auranofin (M=679,5 g/mol) in THF (10 Gew.%) bei RT angesetzt. Diese Lösung wird mit einer zweiten Lösung aus Polylaktid (L210; Fa. Boehringer-Ingelheim) in THF (10 Gew%) derart bei RT gemischt, dass Goldsalz und Polylaktid ein Gew.-Verhältnis von 1:1 aufweisen.

Der Stent wird von Staub und Rückständen gereinigt und in eine geeignete Stentbeschichtungsapparatur (DES Coater, Eigenentwicklung Fa. Biotronik) eingespannt. Mit Hilfe eines Airbrush Systems (Fa. EFD oder Spraying System) wird der sich drehende Stent unter konstanten Umgebungsbedingungen (Raumtemperatur; 42% Luftfeuchte) halbseitig mit der Goldsalz/Polymermischung beschichtet. Bei einem Düsenabstand von 20 mm ist ein 18 mm langer Stent nach ca. 10 min beschichtet. Nach Erreichen der beabsichtigten Schichtmasse wird der Stent 5 min bei Raumtemperatur getrocknet, ehe nach Drehen des Stents und erneutem Einspannen die unbeschichtete Seite auf dieselbe Weise beschichtet wird. Der fertig beschichtete Stent wird für 36 h bei 40°C in einem Vakuumofen (Vakucell; Fa. MMM) getrocknet.

Eine Schichtdicke der aufgetragenen Beschichtung beträgt etwa 3-5 µm.

## Patentansprüche

1. Stent mit einer Beschichtung oder Kavitätenfüllung bestehend aus oder enthaltend einen organischen Au-Komplex.

2. Stent nach Anspruch 1, bei dem der Stent ganz oder in Teilen aus einem biokorrodierbaren metallischen Werkstoff besteht.

3. Stent nach einem der Ansprüche 1 und 2, bei dem der biokorrodierbare metallische Werkstoff eine Magnesiumlegierung ist.

4. Stent nach einem der Ansprüche 1 bis 3, bei dem der Au-Komplex ein Au(I)- oder Au(III)-Komplex ist.

5. Stent nach einem der Ansprüche 1 bis 4, bei dem der Au-Komplex ein Au(I)-Komplex ist und einen oder mehrere tertiäre Phosphinliganden aufweist.

6. Stent nach Anspruch 5, bei dem der Phosphinligand ausgewählt ist aus der Gruppe umfassend Triethylphosphin, 1,2-bis(Diphenylphosphin)ethan, 1,2-bis(Dipyridylphosphin)ethan, und tris(Hydroxymethyl) phosphin.

7. Stent nach einem der Ansprüche 1 bis 4, bei dem der Au-Komplex ein Au(III)-Komplex ist und einen oder mehrere mehrzähnige N-haltige Liganden aufweist.

8. Stent nach Anspruch 7, bei dem der N-haltige Ligand ausgewählt ist aus der Gruppe umfassend Ethylendiamin, Diethylendiamin, N-Benzyl-N,N-dimethylamin; aus Bispyridyl-Liganden, insbesondere (Bispyridyl)(OH)₂, (6-(1,1-Dimethylbenzyl)-2,2'-bipyridin-H)(OH); aus Trichlor(2-pyridylmethanol), Dichlor(N-ethylsalicylaldiminat), Trichlor(diethylendiamin), und Trichlor(bisethylendiamin) oder aus Dithiocarbamat.

9. Stent nach einem der Ansprüche 1 bis 8, bei dem der Au-Komplex ein antikanzerogener oder immunmodulatorischer Wirkstoff ist.

10. Stent nach Anspruch 9, bei dem der Wirkstoff ausgewählt ist aus der Gruppe umfassend Aurothiomalat, Aurothioglucose, Auranofin, bis(Thiosulfat) Au(I), Thiopropansulfat-S-Au(I), Tetraacetyl-P-D Thioglucose Au(I) triethylphosphin, 1,2-bis(Diphenylphosphin)ethan Au(I), 1,2-bis(Dipyridylphosphino)ethan Au(I), Tetrakis ((tris(hydroxymethyl)) phosphin) Au(I) oder Dithiocarbamat Au(III).

11. Stent nach einem der Ansprüche 1 bis 10, bei dem der Au-Komplex in eine polymere organische Matrix eingebettet ist, die als Beschichtung oder Kavitätenfüllung auf dem Stent aufgebracht ist.

12. Verwendung von organischen Au-Komplexen zur Beschichtung oder Kavitätenfüllung von Stents.
